(19) Europäisches Patentamt
European Patent Office
Office européen des brevets

(11) Veröffentlichungsnummer: **0 185 988**
**B1**

# (12) EUROPÄISCHE PATENTSCHRIFT

(45) Veröffentlichungstag der Patentschrift: **16.03.88**

(51) Int. Cl.⁴: **C 07 C 91/23**, C 07 C 93/04, C 07 D 295/08, C 07 C 89/02, A 61 K 31/13

(21) Anmeldenummer: **85115389.0**

(22) Anmeldetag: **04.12.85**

(54) **10,11-Dihydro-5H-dibenzo a,d cycloheptadien-Derivate, ihre Herstellung und Verwendung.**

(30) Priorität: **08.12.84 DE 3444837**

(43) Veröffentlichungstag der Anmeldung: **02.07.86 Patentblatt 86/27**

(45) Bekanntmachung des Hinweises auf die Patenterteilung: **16.03.88 Patentblatt 88/11**

(84) Benannte Vertragsstaaten: **AT BE CH DE FR GB IT LI NL SE**

(56) Entgegenhaltungen:
**EP-A-0 018 076**
**AT-B-342 034**
**DE-A-2 328 758**
**DE-A-3 311 099**

(73) Patentinhaber: **BASF Aktiengesellschaft, Carl-Bosch- Strasse 38, D-6700 Ludwigshafen (DE)**

(72) Erfinder: **Franke, Albrecht, Dr., Mandelring 11, D-6706 Wachenheim (DE)**
Erfinder: **Mueller, Claus, D., Dr., Im Schaflaeger 30, D-6806 Viernheim (DE)**
Erfinder: **Lenke, Dieter, Prof. Dr., Kekuleplatz 1, D-6700 Ludwigshafen (DE)**

**EP 0 185 988 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Die Erfindung betrifft neue 10,11-Dihydro-5H-dibenzo[a,d]cycloheptadien-Derivate, Verfahren zu deren Herstellung sowie deren Verwendung bei der Bekampfung von Krankheiten.

Aus der DE-OS-23 28 758 sind bereits Diphenylmethyl-Derivate der Formel $(C_6H_5)_2CH-CH_2-COH(CH_3)-CH_2-NH-Alkyl$ bekannt, die Wirkungen auf das Zentralnervensystem besitzen und krampflösend wirken. Diese Verbindungen wirken auch antiarrhythmisch.

Es wurde nun gefunden, daß 10,11-Dihydro-5H-dibenzo[a,d]cycloheptadien-Derivate der Formel

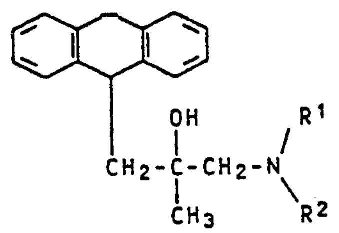 I,

in der

$R^1$ ein Wasserstoffatom oder eine $C_{1-6}$-Alkyl-, $C_{2-6}$-Alkenyl-, $C_{2-6}$-Alkinyl, $C_{1-6}$-Hydroxyalkyl- oder $C_{1-4}$-Alkoxy-$C_{1-4}$-alkylgruppe und

$R^2$ ein Wasserstoffatom oder

$R^1$ und $R^2$ zusammen mit dem sie verbindenden Stickstoffatom einen Morpholinring oder einen Piperidinring, der in der 4-Stellung durch eine Hydroxygruppe und/oder durch eine gegebenenfalls durch ein Halhogenatom substituierte Phenylgruppe substituiert sein kann,

bedeuten, sowie deren Salze mit physiologisch verträglichen Sauren zur Therapie von Herzrhythmusstörungen geeignet sind.

Als Säuren zur Bildung physiologisch verträglicher Salze kommen vorzugsweise Halogenwasserstoffsäuren, wie Bromwasserstoffsäure und insbesondere Chlorwasserstoffsäure, mit der die erfindungsgemäßen Verbindungen besonders gut kristallisierende Salze bilden, in Frage. Ferner sind zu nennen: Phosphorsäure, Salpetersäure, Schwefelsäure, mono- und bifunktionelle Carbonsäuren und Hydroxycarbonsäuren - wie Essigsäure, Oxalsäure, Maleinsäure, Bernsteinsäure, Fumarsäure, Weinsäure, Zitronensäure, Salicylsäure, Sorbinsäure, Milchsäure -, sowie Sulfonsäuren - wie p-Toluolsulfonsäure und 1,5-Naphthalindisulfonsäure -.

Die neuen Verbindungen lassen sich herstellen, indem man 2-[(10,11-Dihydro-5H-dibenzo-cycloheptadienyl)-methyl]-2-methyloxiran mit einem Amin der Formel $HNR^1R^2$, worin $R^1$ und $R^2$ die angegebenen Bedeutungen haben, umsetzt und die so erhaltenen Verbindungen gegebenenfalls in ihre Salze mit physiologisch verträgliche Säuren überführt.

In der Regel wird als Lösungsmittel ein niederer Alkohol, vorzugsweise Ethanol oder n-Propanol, verwendet und die Umsetzung zweckmäßigerweise in der Siedehitze des verwendeten Alkohols durchgeführt. Es können aber auch andere Lösungsmittel, wie Tetrahydrofuran, Dioxan, Acetonitril oder Dimethylformamid verwendet werden.

Nach Beendigung der Reaktion werden die erhaltenen Verbindungen zweckmäßig durch Destillation oder nach der Überführung in ein Säureadditionssalz durch Umkristallisation gereinigt.

Das für die Herstellung der neuen Substanzen benötigte 2-(10,11-Dihydro-5H-dibenzo-cycloheptadienyl)-methyl]-2-methyloxiran läßt sich durch Umsetzen von [(10,11-Dihydro-5H-dibenzo-cycloheptadienyl)-aceton mit Trimethylsulfoxoniumiodid in Gegenwart von Natriumhydroxid in Dimethylsulfoxid herstellen (vgl. J. Amer. Chem. Soc. 84, 3782 (1962)).

Die erfindungsgemäßen Verbindungen fallen bei der Herstellung als Racemate an, die gegebenenfalls durch fraktionierte Kristallisation geeigneter Salze optisch aktiver Säuren in die optischen Antipoden nach an sich üblichen Methoden getrennt werden können.

Die erfindungsgemäßen Verbindungen und ihre physiologisch verträglichen Säureadditionssalze sind wegen ihrer antiarrhythmischen Eigenschaften insbesondere zur Behandlung von Herz-Rhythmusstörungen zur Prophylaxe des plötzlichen Herztodes und zur Behandlung der koronaren Herzkrankheit geeignet. Zur Untersuchung der pharmakodynamischen Eigenschaften der erfindungsgemäßen Produkte wurde die folgende Methode verwendet:

Die Substanzen wurden Ratten (Stamm: Sprague-Dawley, Gewicht: 200 bis 250 g) oral appliziert. 45 min danach wurden die Tiere mit Thiobutabarbital-Na (100 mg/kg i.p.) narkotisiert. Als arrhythmogene Substanz wurde Aconitin 60 min nach Substanzapplikation i.v. (V. Jugularis) infundiert (Dosierungsgeschwindigkeit: 0,005 mg/kg x min). Bei unbehandelten Tieren ($N = 52$) traten nach $2,74 \pm 0,07$ Minuten im EKG Arrhythmien auf, deren Eintritt durch Antiarrhythmika dosisabhängig verzögert wurde.

Aus der linearen Beziehung zwischen log Dosis (mg/kg) der Prufsubstanzen und der relativen Verlangerung der Aconitininfusionsdauer (Δ %) wurde die Dosis bestimmt, welche die Aconitininfusionsdauer um 50 % verlangert (ED 50 %).

Ferner wurde aus der in den Experimenten verwendeten dezimalgeometrischen Dosenfolge (Faktor 2,154) die Dosis ermittelt, bei welcher toxische Symptome (Veränderung im Ausgangs-EKG, Cynose, Krämpfe) auftreten.

Als Maß für die therapeutische Breite der neuen Verbindungen wurde der Quotient aus akuter toxischer Dosis und antiarrhythmisch wirksamer ED 50 % bestimmt.

Die erfindungsgemäßen Verbindungen sind bei oraler Applikation in Dosen unter 43,5 mg/kg antiarrhythmisch wirksam und übertreffen damit die Vergleichßsubstanz Chinidin eindeutig. Darüber hinaus verfügen sie über eine größere therapeutische Breite als die Vergleichsverbindung.

**Tabelle 1**

Antiarrhythmische Wirkung
Ratte; Appl.: per os

| Beispiel | ED 50 % (mg/kg) 1) |
|---|---|
| 1 | 6,51 |
| 9 | 2,57 |
| 3 | 3,11 |
| 4 | 6,47 |
| 6 | 6,83 |
| 5 | 7,78 |
| Chindin | 43,5 |

1) Dosis (mg/kg), welche die Aconitininfusionsdauer um 50 % verlängert.

**Tabelle 2**

Antiarrhythmische Wirkung und Toxizität
Ratte; Appl.: per os

| Beispiel Nr. | ED 50 % 1) (mg/kg) | Toxizität Dosis (mg/kg2) | Q3) |
|---|---|---|---|
| 3 | 3,11 | 100 | 32 |
| 4 | 6,47 | 215 | 33 |
| Chindin | 43,5 | 464 | 11 |

1) Dosis, welche die Aconitininfusionsdauer um 50 % verlängert.
2) Dosis (mg/kg), nach welcher erste toxische Symptome beobachtet werden.
3) $Q = \frac{\text{Toxische Dosis}}{\text{ED 50 \%}}$

Die neuen Verbindungen können in üblicher Weise oral oder parenteral (intravenös, intramuskulär) verabfolgt werden.

Die Dosierung hängt vom Alter, Zustand und Gewicht des Patienten sowie von der Applikationsart ab. In der Regel beträgt die tägliche Wirkstoffdosis zwischen etwä 0,5 und 15 mg/kg Körpergewicht bei oraler Gabe und zwischen etwa 0,005 und 0,1 mg/kg Körpergewicht bei parenteraler Gabe.

Die neuen Verbindungen können in den gebräuchlichen galenischen Applikationsformen fest oder flüssig angewendet werden, z. B. als Tabletten, Filmtabletten, Kapseln, Pulver, Granulate, Dragees, Suppusitorien oder Lösungen. Diese werden in üblicher Weise hergestellt. Die Wirkstoffe können dabei mit den üblichen galenischen Hilfsmitteln wie Tablettenbindern, Füllstoffen, Konservierungsmitteln, Tablettensprengmitteln, Fließreguliermitteln, Weichmachern, Netzmitteln, Dispergiermitteln, Emulgatoren, Lösungsmitteln, Retardierungsmitteln, Antioxidantien und/oder Treibgasen verarbeitet werden (vgl. H. Sucker et al: Pharmazeutische Technologie, Thieme-Verlag, Stuttgart, 1978). Die so erhaltenen Applikationsformen enthalten den Wirkstoff normalerweise in einer Menge von 0,1 bis 99 Gew.-%.

Die folgenden Beispiele erlautern die Erfindung:

A. Herstellung der Ausgangsverbindung

4 g Natriumhydrid (80-%-ig in Paraffinöl) (0,133 mol) werden in 175 ml wasserfreiem Dimethylsulfoxid suspendiert. Unter Rühren und Kuhlen wird bei 15 bis 20°C portionsweise 28,5 g (0,133 moll Trimethylsulfoxoniumiodid hinzugegeben. Nach beendeter Wasserstoffentwicklung wird die Kühlung entfernt und es werden 25 g (0,1 mol) (10,11-Dihydro-5H-dibenzo-cycloheptadienyl)-aceton, gelöst in 80D ml wasserfreiem Dimethylsulfoxid, innerhalb 1 h eingetragen. Anschließend wird noch 2 h bei 40°C gerührt. Die Reaktionslösung wird auf 2 l Eiswasser gegossen, 2 h unter Kühlung gerührt. Anschließend wird mit Diethylether extrahiert und das Extraktionsmittel abdestilliert.

Es werden 25,9 g 2-[(10,11-Dihydro-5H-dibenzo-cycloheptadienyl)-methyl]-2-methyloxiran als Rohprodukt isoliert, welches ohne weitere Reinigung verwendet werden kann.

B. Herstellung der Endprodukte

**Beispiel 1**

7 g 2-[(10,11-Dihydro-5H-dibenzo-cycloheptadienyl)-methyl]-2-methyl-oxiran (0,027 mol) und 4 ml Isopropylamin werden in 50 ml Isopropanol 8 h lang unter Rückfluß erhitzt.

Das Lösungsmittel wird unter vermindertem Druck abdestilliert, der Rückstand in Ether aufgenommen, die Etherlösung mit Wasser gewaschen, anschließend mit $Na_2SO_4$ getrocknet und HCl-Gas bis zur Neutralreaktion eingeleitet. Der gebildete Niederschlag wird abfiltriert und aus Aceton/Ether umkristallisiert.

Es werden so 3,6 g (37 %) N-[2-Hydroxy-2-methyl-3-(10,11-dihydro-5H-dibenzo-cycloheptadienyl)-propyl]-N-isopropylaminohydrochlorid, Fp. 158° C isoliert.

Analog werden die folgenden Verbindungen hergestellt bzw. lassen sich herstellen:

| Beispiel | $R^1$ | $R^2$ | Fp [° C] Hydrochlorid |
|---|---|---|---|
| 2 | -H | H | 192 |
| 3 | $-CH_3$ | H | 207 |
| 4 | $-C(CH_3)_3$ | H | 211 |
| 5 | $-CH_2-CH_2-CH_3$ | H | 169 |
| 6 | $-C(CH_3)-CH_2OCH_3$ | H | 136 |
| 7 | $-(CH_2)_5-$ | | 195 |
| 8 | $-(CH_2)_2-O-(CH_2)_2-$ | | 157 |
| 9 | $-CH_2-CH_2-C-CH_2-CH_2-$ | | 161 |
| | FIGUR 0/1 | | |
| 10 | $-C(CH_3)_2-C{\equiv}CH$ | H | 232 |
| 11 | $-CH_2-CH_3$ | H | |
| 12 | $-CH_2-(CH_2)_2-CH_3$ | H | |
| 13 | $-CH_2-CH(CH_3)-CH_3$ | H | |
| 14 | $-CH_2-(CH_2)_4-CH_3$ | H | |
| 15 | $-CH_2-CH(CH_3)-(CH_2)_2-CH_3$ | H | |
| 16 | $-CH_2-C{\equiv}CH$ | H | |
| 17 | $-CH(CH_3)-C{\equiv}CH$ | H | |
| 18 | $-CH_2-CH_2OH$ | H | |

**Patentansprüche**

1. 10,11-Dihydro-5H-dibenzo[a, d]cycloheptadie-Derivate der Formel I

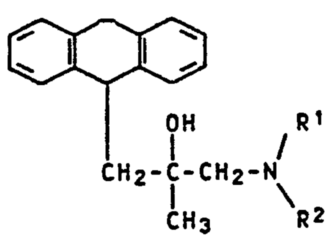

I.

in der

$R^1$ ein Wasserstoffatom oder eine $C_{1-6}$-Alkyl-, $C_{2-6}$-Alkenyl-, $C_{2-6}$-Alkinyl, $C_{1-6}$-Hydroxyalkyl- oder $C_{1-4}$-Alkoxy-$C_{1-4}$-alkyl-gruppe und

$R^2$ ein Wasserstoffatom oder

$R^1$ und $R^2$ zusammen mit dem sie verbindenden Stickstoffatom einen Morpholinring oder einen Piperidinring, der in der 4-Stellung durch eine Hydroxygruppe und/oder durch eine gegebenenfalls durch ein Halogenatom substituierte Phenylgruppe substituiert sein kann,

bedeuten, sowie deren Salze mit physiologisch verträglichen Säuren.

2. Verfahren zur Herstellung der Verbindungen der Formel I gemäß Anspruch 1, dadurch gekennzeichnet, daß man 2-[(10,11-Dihydro-5H-dibenzo-cycloheptadienyl)-methyl]-2-methyloxiran mit einem Amin der Formel $HNR^1R^2$, worin $R^1$ und $R^2$ die angegebenen Bedeutungen haben, umsetzt und die so erhaltenen Verbindungen gegebenenfalls in ihre Salze mit physiologisch verträgliche Säuren überführt.

3. Verbindungen der Formel I gemäß Anspruch 1 zur Verwendung bei der Bekämpfung von Krankheiten.

**Claims**

1. A process for the preparation of a 10,11-dihydro-5H-dibenzo[a,d]cycloheptadiene derivative of the formula

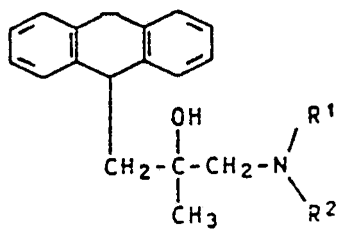

I,

where $R^1$ is hydrogen, $C_1$-$C_6$-alkyl, $C_2$-$C_6$-alkenyl, $C_2$-$C_6$-alkynyl, $C_1$-$C_6$-hydroxyalkyl or $C_1$-$C_4$-alkoxy-$C_1$-$C_4$-alkyl and $R^2$ is hydrogen or $R^1$ and $R^2$ together with the nitrogen atom which connects them to one another, form a morpholine ring or a piperidine ring which may be substituted in the 4-position by hydroxyl and/or by unsubstituted or halogen-substituted phenyl, and its salts with physiologically tolerated acids.

2. A process for the preparation of a compound of the formula I as claimed in claim 1, wherein 2-[(10,11-dihydro-5H-dibenzocycloheptadienyl)-methyl]-2-methyloxirane is reacted with an amine of the formula $HNR^1R^2$, where $R^1$ and $R^2$ have the stated meanings, and, if required, the resulting compound is converted to its salts with physiologically tolerated acids.

3. A compound of the formula I as claimed in claim 1, for use in the treatment of disorders.

**Revendications**

1. Dérivés de 10,11-dihydro-5H-dibenzo (a,d) cycloheptadiène de formule I

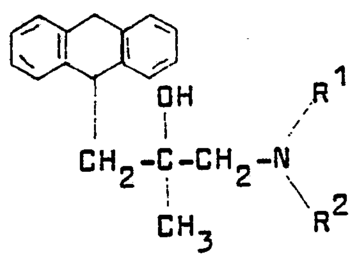

I

dans laquelle

$R^1$ représente un atome d'hydrogène ou un groups alkyle en $C_{1-6}$, alcényle en $C_{2-6}$, alcinyle en $C_{2-6}$, hydroxyalkyle en $C_{1-6}$ ou alcoxy en $C_{1-4}$ - alkyle en $C_{1-4}$, et

$R^2$ représente un atome d'hydrogène ou

$R^1$ et $R^2$ représentent ensemble, avec l'atome d'azote qui les relie, un noyau morpholine ou un noyau piperidine, qui peut être substitué en position-4 par un groupe hydroxy et/ou par un groupe phényle substitué par un atome d'halogène,

ainsi que leurs sels d'acides acceptables physiologiquement.

2. Procédé de préparation de composés de formule I selon la revendication 1 caractérisé par le fait que l'on fait réagir du 2-[(10,11-dihydro-5H-di-benzo-cycloheptadienyl)-methyl]-2-methyloxyranne avec une amine de formule $HNR^1R^2$, où $R^1$ et $R^2$ ont les significations indiquées, et l'on transforme éventuellement les compoaés ainsi obtenus en leurs sels d'acides acceptables physiologiquement.

3. Composés en formule I selon la revendication 1 pour la lutte contre les maladies.